Europäisches Patentamt

European Patent Office

Office européen des brevets

(19)

(11) EP 0 763 525 B1

(12)

# EUROPEAN PATENT SPECIFICATION

(45) Date of publication and mention
of the grant of the patent:
**09.12.1998 Bulletin 1998/50**

(51) Int Cl.⁶: **C07C 303/24**, C07C 305/10,
C11D 1/16

(21) Application number: **96306449.8**

(22) Date of filing: **05.09.1996**

(54) **Detergent manufacture**

Herstellung eines Reinigungsmittels

Fabrication d'un détergent

(84) Designated Contracting States:
**DE ES FR GB IT**

(30) Priority: **14.09.1995 GB 9518860**

(43) Date of publication of application:
**19.03.1997 Bulletin 1997/12**

(73) Proprietors:
• **UNILEVER PLC**
**London EC4P 4BQ (GB)**
Designated Contracting States:
**GB**
• **UNILEVER N.V.**
**3013 AL Rotterdam (NL)**
Designated Contracting States:
**DE ES FR IT**

(72) Inventors:
• **Adams, Amanda Jane**
**Bebington, Wirral, Merseyside, L63 3JW (GB)**
• **Roberts, David William**
**Bebington, Wirral, Merseyside, L63 3JW (GB)**

(74) Representative: **Mole, Peter Geoffrey et al**
**UNILEVER PLC**
**Patent Division**
**Colworth House**
**Sharnbrook**
**Bedford MK44 1LQ (GB)**

(56) References cited:
**WO-A-94/13633**

**Description**

This invention relates to the production of compounds of the general formula:

$$RO(XO)_nSO_3H$$

where R denotes a primary alkyl group and XO denotes an alkylene oxide residue and to the salts of such compounds having the general formula

$$[RO(XO)_nSO_3]_mM$$

where M is a cation having valency m. The parameter n denotes the extent of alkoxylation, and may be zero.

Compounds of the formula $[RO(XO)_nSO_3]_mM$ where n is not zero, are generally known as alkyl ether sulphates (AES) and are used as anionic detergents, provided the cation M is such that they are water-soluble.

Compounds of the formula $RO(XO)_nSO_3H$ are the corresponding acid form.

If n is zero, the compounds are of the formula

$$ROSO_3H$$

and their salt are of the formula

$$[ROSO_3]_mM$$

Such compounds of the formula $[ROSO_3]_mM$ are primary alkyl sulphates, variously known as primary alcohol sulphates. Provided the cation M is such that they are water-soluble, they are anionic detergents.

The compounds of the stated formula $ROSO_3H$ are primary alkyl half esters of sulphuric acid. They are the acid form of primary alkyl sulphate (PAS).

Primary alkyl sulphate and alkyl ether sulphate are customarily produced by sulphation of the corresponding primary alcohol or alkoxylated primary alcohol so as to produce the acid form of the detergent. This acid form is then neutralised to the detergent itself so that the production route is

$$RO(XO)_nH \rightarrow RO(XO)_nSO_3H \rightarrow [RO(XO)_nSO_3]_mM$$

and when n is zero this can be written as

$$ROH \rightarrow ROSO_3H \rightarrow (ROSO_3)_mM$$

A difficulty, however is that the compounds can revert from the acid to the primary alcohol or alkoxylated primary alcohol. The reversion of the acid to starting material is believed to take place by hydrolysis under acidic conditions, leading to the formation of sulphuric acid. It is therefore generally believed that the acid forms of PAS and AES are unstable in aqueous solution. The problem is regarded as particularly acute for PAS acid.

Thus in volume 22 of Kirk-Othmer Encyclopedia of Chemical Technology (1983) the chapter entitled 'Sulphonation and Sulphation' states at page 1 that "sulphated products are unstable toward acid hydrolysis". A similar statement is made on page 25.

De Groot 'Sulphonation Technology in the Detergent Industry' published by Kluwer, Dordrecht 1991 states on page 37 "The product of sulphation of detergent alcohols, i.e. an acid sulphate, is unstable and requires immediate neutralisation". Pages 104 and 105 explain that hydrolysis can commence at a localised acidic region within a quantity of generally alkaline primary alkyl sulphate. Consequently it is taught that a high pH of 9 to 11 must be maintained.

On page 214 the same book states "It is essential to neutralise the acid forms of lauryl alcohol, alcohol ethoxylates and tallow alcohol as quickly as possible to prevent reversion".

To avoid the pitfall of reversion to starting materials, it is normal for the mixture produced in a sulphation reaction to be made to flow directly into a neutralisation process. For instance the reaction mixture coming from a falling film reactor is delivered directly into a neutralisation loop.

In complete contrast to this generally accepted belief, we have found that these acid forms can be stable over significantly extended periods of time in aqueous solution and that this provides a way to manufacture at least some products without the problems associated with decomposition of acid.

Therefore, in a first aspect, the present invention provides a method of producing acid of the formula

$$RO(XO)_nSO_3H$$

where R denotes a straight or branched primary alkyl group containing 8-22 carbon atoms, XO denotes an alkylene oxide residue containing from 2 to 4 carbon atoms and n is an average value which may be zero, comprising sulphating the corresponding primary alcohol of formula

$$RO(XO)_nH$$

and adding the product from the sulphonation reaction to water in such proportions as will at once produce a solution in which the acid of formula

$$RO(XO)_nSO_3H$$

is in either the micellar or lamellar phases.

In a second aspect of the invention provides an aqueous solution of acid of formula $RO(XO)_nSO_3H$ in which the acid is present in micellar or lamellar phase and any cations (apart from hydrogen ions) which are present in the solution are outnumbered by molecules of the said acid.

The above requirement for cations to be outnumbered by molecules of acid is a distinction from mildly acidic detergent compositions containing alkyl ether sulphate or primary alcohol sulphate. In such compositions containing either of these detergents in salt form, the content of cations will outnumber molecules of the corresponding acid.

The micellar and lamellar phases each exist over a respective range of concentrations in water. The boundaries of these concentration ranges are affected by several factors, including

the length of the alkyl chains R,
the proportion of alkyl chains R which are branched,
the extent of alkoxylation, if any,
the presence of other organic or inorganic materials.

In many cases, when other inorganic or organic materials are at low concentrations only, as is usual in detergent manufacture, the micellar phase will exist at concentrations up to 25% by weight, sometimes up to 35% by weight.

It is preferred to generate a solution containing a concentration of 5 to 35% by weight. Suitably, solutions contain from 20 to 35% by weight of acid.

A solution will generally be in the lamellar phase when the acid concentration is in the range from 50% by weight and above, although it is preferred to keep below an upper limit of 85% by weight, and possibly below 75% by weight. It is possible to obtain lamellar phase at concentrations below 50% by weight, possibly down to 35% by weight, if water-soluble inorganic salt such as sodium chloride is present.

When considering concentration of acid, the formula $RO(XO)_nSO_3H$ is taken to include acid in the corresponding ionised form $RO(XO)_nSO_3^-H^+$.

Identification of phases of the acid solutions can be accomplished by examination of the solution under a polarising microscope, just as for detergent solutions.

Dilution of the product of the sulphation reaction with water so as to generate a composition in micellar phase may be carried out by simply running that product into water until a desired concentration of the PAS acid in water is reached. It can be done on a continuous basis by mixing the flow of product from a sulphation reactor with a flow of water, adjusting the flow rate of water to give the desired concentration of acid in the water. By proceeding in this manner, it is practicable to obtain an aqueous solution with an acid content in the range from 5 to 35%s by weight.

Generating a composition which is at once in the lamellar phase can be accomplished by use of a neutralisation loop or similar apparatus. To start up, the loop is charged with an aqueous solution of alkyl or alkyl ether sulphate (or possibly some other detergent) which is at such a concentration that it is in the lamellar phase. If desired, this may be formed in situ in the loop by neutralisation.

The product from a sulphation reaction and water are both then added to the loop, at such rates as will approximately maintain the concentration of solute in the aqueous solution within the loop, and thus maintain lamellar phase within the loop. The neutralised alkyl or alkyl ether sulphate will progressively be discharged from the loop, so that after a period of time the loop will contain the acid in lamellar phase.

Therefore, in one embodiment, the method according to the present invention may be carried out by introducing the product from the sulphation reaction into a circulating loop of solution of the acid and introducing water into the circulating loop in sufficient quantity to maintain the concentration of acid in the loop in a range from 50 to 75% by weight.

In an alternative embodiment, the method according to present invention may be carried out by introducing the product from the sulphation reaction into a circulating loop of lamellar solution of neutralised acid, and introducing water into the circulating loop in sufficient quantity to form a lamellar solution of the acid in the loop while displacing the neutralised acid therefrom.

In a further embodiment, the method according to present invention resulting in acid in the lamellar phase comprises

i) circulating detergent in the lamellar phase around a loop

ii) adding the reaction mixture from sulphation of the alcohol to the loop, and also adding water to the loop so that the lamellar phase is maintained within the loop, while discharging material from the loop, whereby the circulating content of the loop progressively changes to an aqueous solution of the said acid in lamellar phase, and then

iii) containing the addition of said reaction mixture and water, while discharging lamellar phase aqueous acid from the loop.

It is preferred that the sulphation reation and dilution with water are both carried out in a continuous process.

Acid solutions in either micellar or lamellar phase may be kept for some hours, and in certain forms of the invention it is a characteristic that the acid solution is kept for at least 2 hours possibly at least 4 or at least 12 hours prior to neutralisation.

Solutions of acid will normally be neutralised in a subsequent step.

There are several benefits for forming an acidic solution and holding this for a time before neutralisation. Both at relatively high concentrations which will give lamellar phase, and also at lower concentration which will give micellar phase, the formation of free acid, followed by later neutralisation, is advantageous in that it "uncouples" the sulphation and neutralisation steps from each other. Acid can be stored in a holding tank. If it is necessary to stop one or other process temporarily, the other can continue.

Alkyl and alkyl ether sulphates are usually neutralised to their sodium salts. If a small quantity is required, with a different cation, the invention allows this to be provided easily, by taking a portion of the free acid and neutralising with the appropriate base, separately from main production, which might well be carried out using a continuous neutralisation loop.

Dilution of acid to an aqueous solution containing 5 to 35% by weight of acid can prove useful in the production of the magnesium salt form of alkyl or alkyl ether sulphate. Acid of such a concentration can be reacted directly with magnesium oxide.

Dilution of acid to an aqueous solution containing 5 to 35% by weight of acid followed by neutralisation can also be useful when the neutralised aqueous solution is to be used directly in the manufacture of a detergent composition, for instance an aqueous hand dishwashing liquid.

For the present invention, sulphation may be carried out in a conventional manner. Nowadays this is generally carried out using sulphur trioxide and a falling film reactor.

The feedstock for sulphation will be an alcohol or alkoxylated alcohol of formula $RO(XO)_nH$ where n may be zero. The alkyl chain R can be of conventional chain length for detergents, which is from about 8 to 22 carbon atoms, especially 8 to 18 carbon atoms. The alkyl chain may be of biological origin, as in coconut and tallow alcohols, or may be derived from petroleum.

The alkoxylation of alcohols with alkylene oxides is well known. It is usually carried out with ethylene oxide or propylene oxide or a combination of the two. Ethoxylation with ethylene oxide is commercially the most important. The parameter n is an average value. Generally, when not zero it will be at least 0.5, and it may range up to values of 12 or more. Values of n in the range from 0.5 to 7 are commonly used. The invention may be of particular interest when n is no greater than 2.

Another advantage of this invention, which arises when the value of n is positive and XO denotes an ethylene oxide residue, is that the proportion of dioxan impurity has been observed to remain at an acceptably low level during storage of the acid.

It is of course envisaged that this invention will be put into practice on an industrial scale. Consequently, when the acid form of alkyl or alkyl ether sulphate is manufactured by means of the invention, the resulting acid is likely to be in quantities of at least 50 Kg, probably at least 200 Kg and frequently at least 1 tonne.

The use of a neutralisation loop to form a lamellar phase aqueous solution of acid will now be described with reference to the accompanying diagrammatic drawing.

This shows a conventional falling film reactor 10 connected via an air separator 12 to a neutralisation loop 14 having heat exchanger 18 and a circulating pump 16 which also functions as a mixer. An outlet from the neutralisation loop is provided at 20.

The material from the falling film reactor 10 enters the loop at the pump 16 which is also provided with an inlet 26 for water.

Neutralisation loops are known per se and the loop which is used may be in accordance with conventional construction of such a loop. An inlet 24 for sodium hydroxide solution is not required for the invention but is available for use during operation of the loop in known manner to form neutralised detergent paste.

The conventional procedure for the continuous production of sodium primary alkyl (or alkyl ether) sulphate in such a loop is to introduce the sulphated reaction product from the falling film reactor 10 into the neutralisation loop which already contains a circulating mass of sodium primary alkyl or alkyl ether sulphate solution containing approximately 70% by weight of the detergent sulphate. Concentrated sodium hydroxide solution and some water are also added to the loop through respective inlets 24, 26 so that neutralisation takes place in the loop with the existing solution in the loop serving to maintain the concentration and act as a diluent for the neutralisation reaction. Product is withdrawn from the loop via the outlet 20 at such a rate that the quantity of circulating material in the loop remains approximately constant.

In order to obtain a lamellar phase solution of the acid, without passing through the viscous cubic phase which exists at concentrations intermediate between the micellar and lamellar phases, the loop is first charged with neutralised alkyl or alkyl ether sulphate circulating as lamellar phase. (This may be present from conventional operation of the loop, or may come from another source and be loaded into the loop.)

The loop is put into operation, receiving reaction mixture from the falling film reactor 10, and water via inlet 26. There is no admission of sodium hydroxide. Any previous supply of sodium hydroxide solution arriving via inlet 24 is turned off. The result is that the sulphated reaction product from the reactor is diluted with water but is not neutralised. The amount of water added via inlet 26 is set such that the concentration of solute in the aqueous solution circulating in the loop remains high. As material is withdrawn from the loop, the composition of the material circulating within the loop and withdrawn through the outlet 20 progressively changes from fully neutralised sulphate to a partially neutralised mixture of sulphate and acid, then eventually to an aqueous solution of the acid, at a concentration which exists as a lamellar phase.

During the change over, the concentration of solute in the loop may be adjusted from a concentration at which neutralised sulphate is in lamellar phase (e.g. 70% to a concentration at which the acid is in lamellar phase (e.g. 65%). This can be achieved by regulating the supply of water.

By means of this apparatus and procedure it is possible to dilute the reaction product from a falling film reactor to form a concentrated aqueous solution of PAS acid without the transient formation of PAS acid solutions with a concentration in the range from about 40 to 55% by weight. Such solutions can be expected to be in the very viscous cubic phase (absent inorganic salt or other expedient to achieve lamellar phase at concentration below 50%) like solutions of sodium primary alkyl sulphate of the same concentration range.

A more dilute solution of acid, having a concentration of 5 to 35% by weight, may be produced in a similar loop. However, there will never be any need for sodium hydroxide admission and the loop can contain water at start up.

Example 1

A commercially available fatty alcohol having carbon chain lengths in the range from 12 to 15 carbon atoms (Lial 125 available from Enichem) was sulphated continuously on a multi-tube 6 metre falling film reactor and then neutralised conventionally in a neutralisation loop to produce an aqueous paste of sodium primary alkyl sulphate. This sulphation and neutralisation was carried out as normal commercial production. The aqueous paste of primary alkyl sulphate was found by analysis to have a non-detergent organic matter content (NDOM) of 1.5% by weight based on the quantity of sodium primary alkyl sulphate.

During the course of sulphation a batch of primary alkyl sulphuric acid was collected from the base of the falling film reactor and added with stirring to water at room temperature so as to obtain a solution containing 30% by weight of primary alkyl sulphuric acid in water. The solution was clear, mobile and pale straw coloured at ambient temperature. After various intervals of time samples of this solution were neutralised with aqueous sodium hydroxide solution and the neutralised samples were analysed.

It was found that the NDOM content as percentage of the amount of sodium primary alkyl sulphate was 2.3% by weight after one hour and 2.9% by weight after 22.5 hours. Comparing these figures with the value of 1.5% NDOM for the sodium primary alkyl sulphate produced by immediate neutralisation in the loop it can be seen that there was very little decomposition of the primary alkyl sulphuric acid in solution over a period of 22.5 hours.

Example 2

A commercially available fatty alcohol (LIAL 123 available from Enichem) was sulphated continuously on a pilot scale 2.5 metre single tube falling film reactor. The resulting PAS acid was then fed into a neutralisation loop, together with sodium hydroxide and water. The feed rates were adjusted to produce a neutralised paste of about 65% by weight PAS.

Then the supply of sodium hydroxide to the loop was stopped and the water feed rate was adjusted, so as to give acid pastes of various concentrations. Viscosity increases during the transition from neutralised to acid paste were observed but were not sufficient to cause handling problems during the run. Two acid pastes were collected. They were stored at ambient temperature and samples were neutralised and analysed at various time intervals.

Measurements of acid concentration were taken immediately and after 1, 9 and 21 days.

The results are shown in Table 1.

Table 1

| Acid input to loop, ca 13.5 kg/hr | | |
|---|---|---|
| Nominal water input to loop | 10 kg/hr | 6 kg/hr |
| Theoretical % of PAS acid in the acid paste | 57.45 | 69.23 |
| % PAS acid measured, t=:0 | 54.9 | 60.8 |
| 1 day | 49.7 | 49.9 |
| 9 days | 47.6 | 47.2 |
| 21 days | 39.7 | 39.6 |

From Table 1 it is evident that with a nominal water input of 10 kg/hr a 55% PAS acid paste was obtained with little if any decomposition during paste manufacture in the loop - the combined contribution from decomposition and conversion shortfall is only ca.4%, which is not significantly greater than the conversion shortfall normally experienced in alcohol sulphation in a typical reactor of the type used in this example.

With a nominal water input of 6 kg/hr, corresponding to a theoretical PAS acid value of 69%, an acid paste was obtained with a PAS acid value of 61%, with evidence of some decomposition during paste manufacture in the loop.

Rate constants were obtained for the decomposition of the acid pastes on storage. These did not vary greatly. From these rate constants it can be calculated that the pastes were stable over a period of several hours, but not over a prolonged period of storage.

Example 3

Coconut alcohol (Nafol 1218 from Condea, having a molecular weight in the range 204-216) was sulphated on a 6 metre multi-tube falling film reactor. A sample (145 gms) of the mixture from the reactor was collected from the base of the reactor and mixed with water (330 gms) at 35-40°C. The resulting acid concentration was approximately 28% by weight.

The solution obtained was clear and mobile, and did not show evidence of separation or precipitation on overnight storage at ca 20°C.

Samples were neutralised and analysed for AD after 16 hr and 40 hr at ca 20°C. No significant decomposition of the PAS acid was observed.

Example 4

Another sample of mixture from the reactor (as in Example 3, but 91.8 gms) was mixed with water (280.5 gms) at 35-40°C. The resulting acid concentration was approximately 23% by weight acid. 41 parts by weight of this paste were neutralised by dissolving 19.25 parts by weight of magnesium oxide in it, so as to make a magnesium PAS paste containing a substantial excess of magnesium oxide. Analysis of this paste gave an AD value of 16.1, in close agreement with the theoretical value of 16.6 calculated on the basis of the weights of materials used.

Example 5

Ethoxylated alcohol of the formula

$$RO(C_2H_4O)_nH$$

where R denotes $C_{12}$ and $C_{13}$ alkyl chains and n has an average value of 1 (Lialet 123-1 from Enichem) was sulphonated on a 6 metre multi-tube falling film reactor.

A sample of the mixture from the base of the reactor was collected and mixed with water in the proportions by weight of 98.8 parts of acid to 371 parts of water, corresponding to an acid concentration of about 20% by weight acid.

The resulting clear mobile liquid was stored at ambient temperature (ca 20°C). Samples were neutralised at various time intervals and subsequently analysed for acid and for non-detergent organic matter NDOM). The results are shown in Table 2.

Table 2

| t (hr) | LES acid % | NDOM (% of acid) | Dioxan (ppm, Based on acid) |
|---|---|---|---|
| 1 | 4.86 | 3.48 | <25 |
| 3 | 6.75 | 3.35 | <25 |
| 5 | 5.55 | 3.37 | <25 |
| 24 | 6.91 | 3.57 | <25 |
| 48 | 4.55 | 3.71 | <25 |

The NDOM percentage, based on 100% LES acid, did not vary significantly over the whole storage period of 48 hr, and was in the range of the normal LES specification. The dioxan proportion remained at a satisfactory low level throughout this time. It is clear that this acid does not undergo significant hydrolysis over a time period of 48 hr at 20°C.

Example 6

A sample of the solution of Example 5 was used, within a few minutes of its manufacture, by dissolving 16.39 parts by weight of magnesium oxide in 65.77 parts by weight of acid solution. The LES-1 paste, containing a substantial excess of magnesium oxide, had an LES acid value of 19.7%, somewhat higher than the theoretical value, calculated on the basis of the weights of materials used, of 16.5%. The discrepancy may have resulted from settling out of some of the excess magnesium oxide during sampling.

Result was a mobile solution of detergent of formula $[RO(C_2H_4O)]_2$ Mg containing dispersed excess magnesium oxide.

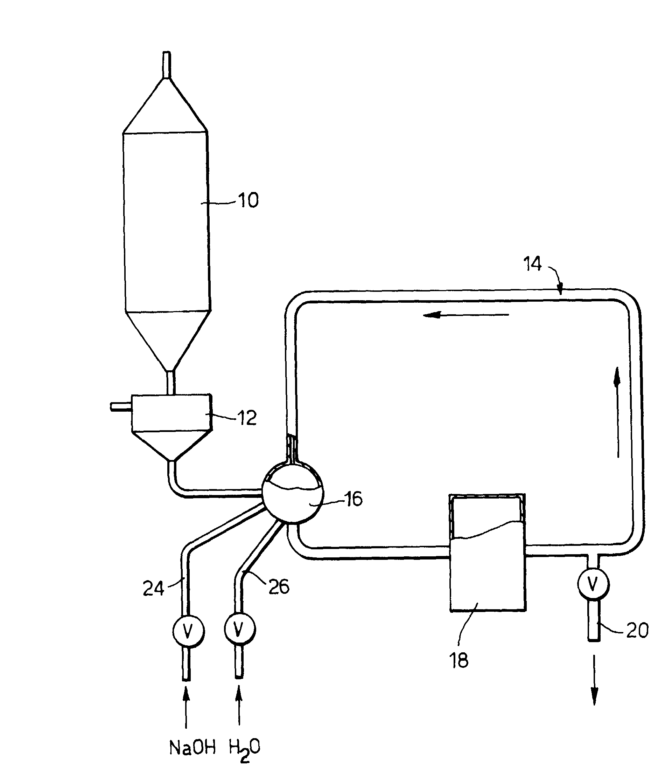

**Claims**

1. A method of producing an acid of the formula

$$RO(XO)_nSO_3H$$

where R denotes a straight or branched primary alkyl group containing 8 to 22 carbon atoms, XO denotes an alkylene oxide residue containing from 2 t 4 carbon atoms and n is an average value which may be zero, comprising sulphating the corresponding primary alcohol of formula

$$RO(XO)_nH$$

and diluting the product from the sulphation reaction with water so as to produce a solution in which the said acid is in either the micellar or lamellar phases.

2. A method according to claim 1 wherein sulphation and dilution are both carried out continuously.

3. A method according to claim 1 or claim 2 wherein the resulting solution is in micellar phase and contains from 5 to 35% by weight of acid.

4. A method according to claim 3 wherein the resulting solution contains from 20 to 35% by weight of acid.

5. A method according to claim 2 wherein the resulting solution is in lamellar phase and contains from 50 to 85% by weight of acid.

**6.** A method according to any one of the preceding claims carried out by introducing the product from the sulphation reaction into a circulating loop of solution of the acid and introducing water into the circulating loop in sufficient quantity to maintain the concentration of acid in the loop in a range from 50 to 75% by weight.

**7.** A method according to claim 5 carried out by introducing the product from the sulphation reaction into a circulating loop of lamellar solution of neutralised acid, and introducing water into the circulating loop in sufficient quantity to form a lamellar solution of the acid in the loop while displacing the neutralised acid therefrom.

**8.** A method according to any one of claims 1 to 7 further comprising a step of neutralising the solution with a base in which the cation is other than sodium.

**9.** A method according to any one of the preceding claims wherein the acid is kept for at least 4 hours before neutralisation.

**10.** A method according to any one of the preceding claims wherein the acid is produced in a quantity of at least 50 kg.

**11.** A method according to anyone of the preceding claims wherein n is zero so that the acid is of formula $ROSO_3H$ or alternatively wherein n has a value in the range 0.5 to 7.

**12.** A method according to claim 1 wherein the resulting acid is in lamellar phase, which method comprises

    i) circulating detergent in the lamellar phase around a loop,
    ii) adding the reaction mixture from sulphation of the alcohol to the loop, and also adding water to the loop so that the lamellar phase is maintained within the loop, while discharging material from the loop, whereby the circulating content of the loop progressively changes to an aqueous solution of the said acid in lamellar phase, and then
    iii) continuing the addition of said reaction mixture and water, while discharging lamellar phase aqueous acid from the loop.

**13.** An aqueous solution of acid of formula $RO(XO)_nSO_3H$ in which the acid is present in micellar or lamellar phase and any cations (apart from hydrogen ions) which are present in the solution are outnumbered by molecules of the said acid wherein R, XO and n are as defined in claim 1.

**14.** An aqueous solution according to claim 13 which is in micellar phase and containing from 5% to 35% by weight of the acid.

**15.** An aqueous solution according to claim 14 containing from 20 to 35% by weight of the acid.

**16.** An aqueous solution according to claim 13 which is in lamellar phase and contains 35% to 85% by weight of acid.

**17.** An aqueous solution according to claim 13 which is in lamellar phase and contains 50% to 85% by weight of acid.

**18.** An aqueous solution according to any one of claims 13 to 17 in a quantity of at least 50 Kg.


**Patentansprüche**

**1.** Verfahren zur Herstellung einer Säure der Formel

$$RO(XO)_nSO_3H$$

    worin R eine gerad- oder verzweigtkettige primäre Alkylgruppe mit 8 bis 22 Kohlenstoffatomen bedeutet, XO einen Alkylenoxidrest mit 2 bis 4 Kohlenstoffatomen bedeutet und n einen Mittelwert darstellt, der null sein kann, umfassend Sulfieren des entsprechenden primären Alkohols der Formel

$$RO(XO)_nH$$

und Verdünnen des Produkts aus der Sulfierung mit Wasser, so daß eine Lösung hergestellt wird, in der die Säure in micellarer oder lamellarer Phase vorliegt.

2. Verfahren nach Anspruch 1, wobei sowohl Sulfieren als auch Verdünnen kontinuierlich ausgeführt werden.

3. Verfahren nach Anspruch 1 oder Anspruch 2, wobei die erhaltene Lösung in micellarer Phase vorliegt und 5 bis 35 Gew.-% Säure enthält.

4. Verfahren nach Anspruch 3, wobei die erhaltene Lösung 20 bis 35 Gew.-% Säure enthält.

5. Verfahren nach Anspruch 2, wobei die erhaltene Lösung in lamellarer Phase vorliegt und 50 bis 85 Gew.-% Säure enthält.

6. Verfahren nach einem der vorangehenden Ansprüche, ausgeführt durch Einführung des Produktes aus der Sulfierung in eine Kreislaufschlaufe der Lösung der Säure und Einführen von Wasser in die Kreislaufschlaufe in ausreichender Menge, um die Konzentration der Säure in der Schlaufe im Bereich 50 bis 75 Gew.-% beizubehalten.

7. Verfahren nach Anspruch 5, ausgeführt durch Einführen des Produkts aus der Sulfierung in eine Kreislaufschlaufe der lamellaren Lösung von neutralisierter Säure und Einführen von Wasser in die Kreislauf schlaufe in ausreichender Menge, um eine lamellare Lösung der Säure in der Schlaufe zu erzielen, während die neutralisierte Säure daraus verdrängt wird.

8. Verfahren nach einem der Ansprüche 1 bis 7, das außerdem einen Schritt der Neutralisierung der Lösung mit einer Base umfaßt, wobei das Kation nicht Natrium ist.

9. Verfahren nach einem der vorangehenden Ansprüche, wobei die Säure vor der Neutralisation für mindestens 4 Stunden gehalten wird.

10. Verfahren nach einem der vorangehenden Ansprüche, wobei die Säure in einer Menge von mindestens 50 kg hergestellt wird.

11. Verfahren nach einem der vorangehenden Ansprüche, wobei n null ist, so daß die Säure die Formel $ROSO_3H$ aufweist, oder alternativ, wobei n einen Wert im Bereich 0,5 bis 7 aufweist.

12. Verfahren nach Anspruch 1, wobei die erhaltene Säure in lamellarer Phase vorliegt, wobei das Verfahren umfaßt

i) im Kreislauf führen des Waschmittels in der lamellaren Phase um eine Schlaufe,
ii) Zugabe des Reaktionsgemisches aus der Sulfierung des Alkohols zu der Schlaufe, und ebenfalls Zugabe von Wasser zu der Schlaufe, so daß die lamellare Phase innerhalb der Schlaufe beibehalten wird, während das Material aus der Schlaufe abgelassen wird, wodurch der im Kreislauf geführte Anteil der Schlaufe sich zunehmend zu einer wässerigen Lösung der Säure in der lamellaren Phase ändert, und dann
iii) Fortführen der Zugabe des Reaktionsgemisches und Wasser, während lamellare Phase-wässerige Säure aus der Schlaufe abgelassen wird.

13. Wässerige Lösung der Säure der Formel $RO(XO)_nSO_3H$, wobei die Säure in micellarer oder lamellarer Phase vorliegt und beliebige Kationen (außer Wasserstoffionen), die in der Lösung vorliegen, durch die Moleküle der Säure, worin R, XO und n wie in Anspruch 1 definiert sind, übertroffen werden.

14. Wässerige Lösung nach Anspruch 13, die in micellarer Phase vorliegt und 5 bis 35 Gew.-% der Säure enthält.

15. Wässerige Lösung nach Anspruch 14, die 20 bis 35 Gew.-% der Säure enthält.

16. Wässerige Lösung nach Anspruch 13, die in lamellarer Phase vorliegt und 35 bis 85 Gew.-% der Säure enthält.

17. Wässerige Lösung nach Anspruch 13, die in lamellarer Phase vorliegt und 50 bis 85 Gew.-% Säure enthält.

**18.** Wässerige Lösung nach einem der Ansprüche 13 bis 17, in einer Menge von mindestens 50 kg.

## Revendications

**1.** Procédé de production d'un acide de formule:

$$RO(XO)_nSO_3H$$

dans laquelle R représente un groupe alkyle primaire droit ou ramifié contenant 8 à 22 atomes de carbone, XO représente un résidu oxyde d'alkylène contenant de 2 à 4 atomes de carbone et n est une valeur moyenne qui peut être zéro, comprenant la sulfatation de l'alcool primaire correspondant de formule

$$RO(XO)_nH$$

et la dilution du produit issu de la réaction de sulfatation avec de l'eau de façon à produire une solution dans laquelle ledit acide est en phase soit micellaire soit lamellaire.

**2.** Procédé selon la revendication 1, dans lequel on effectué continuellement la sulfatation et la dilution.

**3.** Procédé selon la revendication 1 ou la revendication 2, dans lequel la solution résultante est en phase micellaire et contient de 5 à 35% en poids d'acide.

**4.** Procédé selon la revendication 3, dans lequel la solution résultante contient de 20 à 35% en poids d'acide.

**5.** Procédé selon la revendication 2, dans lequel la solution résultante est en phase lamellaire et contient de 50 à 85% en poids d'acide.

**6.** Procédé selon l'une quelconque des revendications précédentes, effectué en introduisant le produit issu de la réaction de sulfatation dans une boucle de circulation de solution de l'acide et en introduisant de l'eau dans la boucle de circulation en quantité suffisante pour maintenir la concentration d'acide dans la boucle dans une gamme de 50 à 75% en poids.

**7.** Procédé selon la revendication 5, effectué en introduisant le produit issu de la réaction de sulfatation dans une boucle de circulation de solution lamellaire d'acide neutralisé, et en introduisant de l'eau dans la boucle de circulation en quantité suffisante pour former une solution lamellaire de l'acide dans la boucle tout en déplaçant l'acide neutralisé.

**8.** Procédé selon l'une quelconque des revendications 1 à 7, comprenant de plus un stade de neutralisation de la solution avec une base, le cation étant autre que sodium.

**9.** Procédé selon l'une quelconque des revendications précédentes, dans lequel on conserve l'acide pendant au moins 4 heures avant neutralisation.

**10.** Procédé selon l'une quelconque des revendications précédentes, dans lequel on produit l'acide en une quantité d'au moins 50 kg.

**11.** Procédé selon l'une quelconque des revendications précédentes, dans lequel n est zéro de sorte que l'acide est de formule $ROSO_3H$ ou en variante, n a une valeur dans la gamme de 0,5 à 7.

**12.** Procédé selon la revendication 1, dans lequel l'acide résultant est en phase lamellaire, ce procédé comprenant :

i) la circulation d'un détergent en phase lamellaire autour d'une boucle,
ii) l'addition du mélange de réaction issu de la sulfatation de l'alcool à la boucle et également l'addition d'eau à la boucle de sorte que la phase lamellaire se maintient dans la boucle, tout en déchargeant la matière de la boucle, de sorte que le contenu de circulation de la boucle se transforme progressivement en une solution aqueuse dudit acide en phase lamellaire et, ensuite,

iii) la continuation de l'addition dudit mélange de réaction et de l'eau, tout en déchargeant l'acide aqueux en phase lamellaire de la boucle.

13. Solution aqueuse d'acide de formule $RO(XO)_nSO_3H$, l'acide étant présent en phase micellaire ou lamellaire et les cations (abstraction faite des ions hydrogène) qui sont présents dans la solution étant dépassés en nombre par les molécules dudit acide, R, XO et n étant comme défini dans la revendication 1.

14. Solution aqueuse selon la revendication 13, qui est en phase micellaire et qui contient de 5 à 35% en poids de l'acide.

15. Solution aqueuse selon la revendication 14, contenant de 20 à 35% en poids de l'acide.

16. Solution aqueuse selon la revendication 13, qui est en phase lamellaire et qui contient 35 à 85% en poids d'acide.

17. Solution aqueuse selon la revendication 13, qui est en phase lamellaire et qui contient 50 à 85% en poids d'acide.

18. Solution aqueuse selon l'une quelconque des revendications 13 à 17, en une quantité d'au moins 50 kg.

NaOH  H₂O